(19) Europäisches Patentamt
European Patent Office
Office européen des brevets

(11) Veröffentlichungsnummer: **0 105 166**
**B1**

(12) # EUROPÄISCHE PATENTSCHRIFT

(45) Veröffentlichungstag der Patentschrift: **22.04.87**

(21) Anmeldenummer: **83108249.0**

(22) Anmeldetag: **22.08.83**

(51) Int. Cl.⁴: **C 07 D 249/08,** C 07 D 233/60, C 07 D 233/61, C 07 D 231/12, C 07 D 403/06, A 61 K 31/41, A 61 K 31/415

(54) **Substituierte tert.-Butanol-Derivate, Verfahren zu ihrer Herstellung und diese enthaltende antimykotische Mittel.**

(30) Priorität: **02.09.82 DE 3232647**

(43) Veröffentlichungstag der Anmeldung:
**11.04.84 Patentblatt 84/15**

(45) Bekanntmachung des Hinweises auf die Patenterteilung:
**22.04.87 Patentblatt 87/17**

(84) Benannte Vertragsstaaten:
**AT BE CH DE FR GB IT LI NL SE**

(56) Entgegenhaltungen:
**EP - A - 0 040 345**
**DD - A - 146 824**
**DE - A - 3 011 258**

(73) Patentinhaber: **BAYER AG, Konzernverwaltung RP Patentabteilung, D-5090 Leverkusen 1 Bayerwerk (DE)**

(72) Erfinder: **Regel, Erik, Dipl.-Ing., Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**

**EP 0 105 166 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte tert.-Butanol-Derivate, mehrere Verfahren zu ihrer Herstellung und diese enthaltende antimykotische Mittel.

Es ist bereits bekannt geworden, dass Hydroxypropyl-imidazole, wie beispielsweise 2-(4-Biphenylyl)-1-(2,4-dichlorphenyl)-3-(imidazol-1-yl)-2-propanol [DE-OS 2 820 489] bzw. 1-Hydroxymethyl-azol-Derivate [«DE-OS 3 018 865], wie beispielsweise 2-(4-Chlorphenoxymethyl)-3,3-dimethyl-1-(imidazol-1-yl)-butan-2-ol oder 2-(2-Methylphenoxymethyl)-3,3-dimethyl-1-(1,2,4-triazol-1-yl)- und -(imidazol-1-yl)-butan-2-ol, bzw. Diazolyl-alkanole [EP-44 605], wie beispielsweise 1,3-Di-(imidazol-1-yl)-2-(4-chlorphenyl)-2-propanol, gute antimykotische Eigenschaften aufweisen.

Es wurden neue substituierte tert.-Butanol-Derivate der allgemeinen Formel (I)

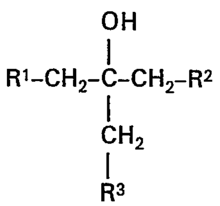

(I)

in welcher

$R^1$ und $R^2$ gleich oder verschieden sind und für Imidazol-1-yl, 1,2,4-Triazol-1-yl; 1,2,4-Triazol-4-yl oder Pyrazol-1-yl stehen, und

$R^3$ für jeweils gegebenenfalls einfach oder mehrfach, gleich oder verschieden substituiertes Phenoxy, Phenylthio, Phenylamino oder Phenyl-N-alkyl-amino mit 1 bis 4 Kohlenstoffatomen im Alkylteil steht, wobei als Phenylsubstituenten jeweils infrage kommen: Halogen, Alkyl mit 1 bis 4 Kohlenstoffatomen, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 5 gleichen oder verschiedenen Halogenatomen wie Fluor- und Chloratomen, Cycloalkyl mit 5 oder 6 Kohlenstoffatomen, Nitro, Cyano, Hydroxycarbonyl, Alkylcarbonyl oder Alkoxycarbonyl mit jeweils 1 bis 4 Kohlenstoffatomen im Alkylteil, gegebenenfalls einfach oder mehrfach, gleich oder verschieden durch Halogen, Nitro, Trifluormethyl und Alkyl, mit 1 bis 2 Kohlenstoffatomen substituiertes Phenyl oder Phenoxy, die Aldehydgruppe sowie der Oxim- bzw. Oximether-Rest

und deren physiologisch verträgliche Säureadditions-Salze gefunden.

Weiterhin wurde gefunden, dass man die substituierten tert.-Butanol-Derivate der Formel (I) erhält, wenn man ein Nucleophil der Formel (II)

$R^1$–H (II)

in welcher

$R^1$ die oben angegebene Bedeutung hat,

a) mit 2-Halogenmethyloxiranen der Formel (IV)

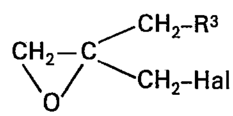

(IV)

in welcher

$R^3$ die oben angegebene Bedeutung hat, und Hal für Halogen steht, in Gegenwart eines Verdünnungsmittels und in Gegenwart eines Säurebindemittels umsetzt, oder

b) mit substituierten Oxiranen der Formel (V)

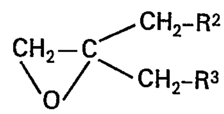

(V)

in welcher

$R^2$ und $R^3$ die oben angegebene Bedeutung haben, in Gegenwart eines Verdünnungsmittels und in Gegenwart eines Säurebindemittels umsetzt.

An die so erhaltenen Verbindungen der Formel (I) kann gegebenenfalls anschliessend eine Säure addiert werden.

Die Verbindungen der Formel (I) besitzen gegebenenfalls ein asymmetrisches Kohlenstoffatom und können deshalb gegebenenfalls in den beiden optischen Isomerenformen anfallen.

Die neuen substituierten tert.-Butanol-Derivate der Formel (I) weisen starke antimykotische Eigenschaften auf. Dabei zeigen überraschenderweise die erfindungsgemässen Verbindungen eine bessere, insbesondere therpaeutisch nutzbare in-vivo-Wirksamkeit als die aus dem Stand der Technik bekannten Verbindungen 2-(4-Biphenylyl)-1-(2,4-dichlorphenyl)-3-(imidazol-1-yl)-2-propanol; 2-(4-Chlorphenoxymethyl)-3,3-dimethyl-1-(imidazol-1-yl)-butan-2-ol; 2-(2-Methylphenoxymethyl)-3,3-dimethyl-1-(1,2,4-triazol-1-yl)- und -(imidazol-1-yl)-butan-2-ol sowie 1,3-Di-(imidazol-1-yl)-2-(4-chlorphenyl)-2-propanol, welches konstitutionell und wirkungsmässig naheliegende Verbindungen sind. Die erfindungsgemässen Stoffe stellen somit eine Bereicherung der Pharmazie dar.

Ausserdem sind die neuen substituierten tert.-Butanol-Derivate interessante Zwischenprodukte. So können z.B. die Verbindungen der allgemeinen Formel (I) an der Hydroxy-Gruppe in üblicher Weise in die entsprechenden Ether überführt werden. Weiterhin können durch Umsetzung mit z.B. Acylhalogeniden oder Carbamoylchloriden in prinzipiell bekannter Weise Acyl- oder Carbamoyl-Derivate der Verbindungen der allgemeinen Formel (I) erhalten werden.

Die erfindungsgemässen substituierten tert.-Butanol-Derivate sind durch die Formel (I) allgemein definiert.

Besonders bevorzugt sind diejenigen Verbindungen der Formel (I), in denen

A) $R^1$ und $R^2$ gleich oder verschieden sind und für Imidazol-1-yl; 1,2,4-Triazol-1-yl; 1,2,4-Triazol-4-yl oder Pyrazol-1-yl stehen, und

$R^3$ für jeweils gegebenenfalls einfach bis dreifach, gleich oder verschieden substituiertes Phenoxy, Phenylthio, Phenylamino oder Phenyl-N-alkyl-amino mit 1 bis 2 Kohlenstoffatomen im Alkylteil steht, wobei als Phenylsubstituenten jeweils infrage kommen: Fluor, Chlor, Brom, Methyl, Ethyl, Isopropyl, tert.-Butyl, 2-Methyl-but-2-yl, Methoxy, Ethoxy, Methylthio, Trifluormethylthio, Cyclohexyl, Nitro, Cyano, Hydroxycarbonyl, Methylcarbonyl, Methoxycarbonyl, Ethoxycarbonyl, jeweils gegebenenfalls einfach bis dreifach, gleich oder verschieden durch Chlor, Nitro, Trifluormethyl oder Methyl substituiertes Phenyl oder Phenoxy, die Aldehydgruppe, Hydroxyiminoethyl und Methoxyiminoethyl.

Im einzelnen seien ausser den bei den Herstellungsbeispielen genannten Verbindungen die folgenden Verbindungen der allgemeinen Formel (I) genannt:

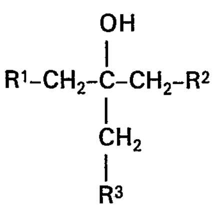

(I)

| $R^1$ | $R^2$ | $R^3$ |
|---|---|---|
| 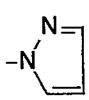 | 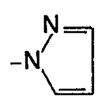 | 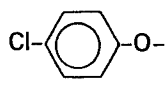 |
| 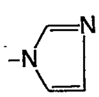 | 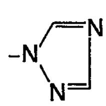 | 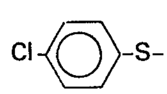 |
| 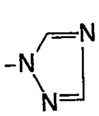 | 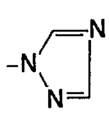 | 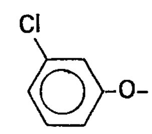 |
| 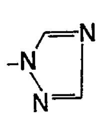 | 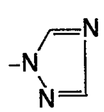 | 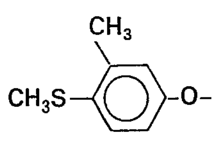 |
| 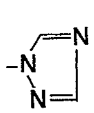 | 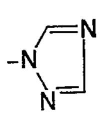 | 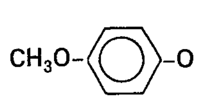 |
| 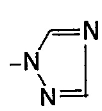 | 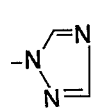 | 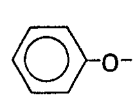 |
| 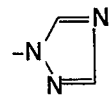 | 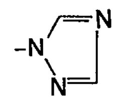 | 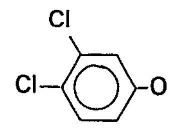 |
| 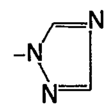 | 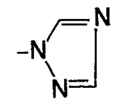 | 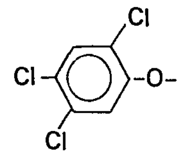 |
| 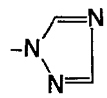 | 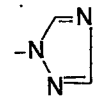 | 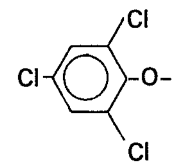 |
| 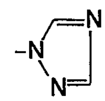 | 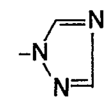 | 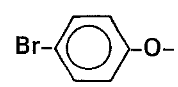 |
| 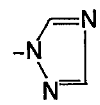 | 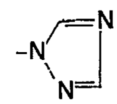 | 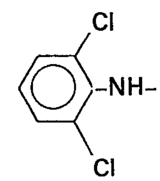 |

Verwendet man beispielsweise 2-Chlormethyl-2-(4-chlorphenoxymethyl)-oxiran und 1,2,4-Triazol als Ausgangsstoffe, so kann der Reaktionsablauf des erfindungsgemässen Verfahrens (a) durch das folgende Formelschema wiedergegeben werden:

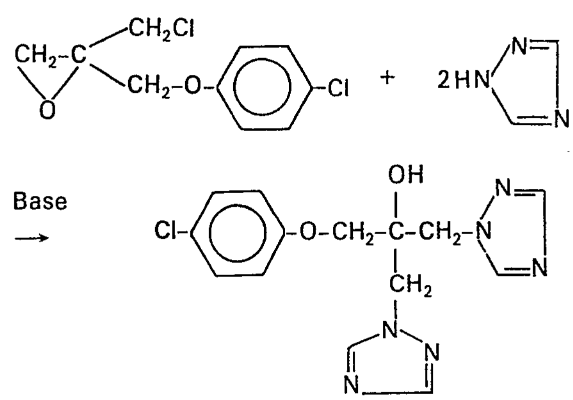


Die für die erfindungsgemässen Verfahren (a) und (b) als Ausgangsstoffe zu verwendenden Nucleophile sind durch die Formel (II) allgemein definiert. In dieser Formel steht $R^1$ vorzugsweise für diejenigen Reste, die bereits im Zusammenhang mit der Beschreibung der erfindungsgemässen Stoffe der Formel (I) vorzugsweise für diesen Substituenten genannt wurden.

Die Nucleophile der Formel (II) sind allgemein bekannte Verbindungen der organischen Chemie.

Die ausserdem für das erfindungsgemässe

Verfahren (a) als Ausgangsstoffe zu verwendenden 2-Halogenmethyloxirane sind durch die Formel (IV) allgemein definiert. In dieser Formel steht $R^3$ vorzugsweise für diejenigen Reste, die bereits im Zusammenhang mit der Beschreibung der erfindungsgemässen Stoffe der Formel (I) vorzugsweise für diesen Substituenten genannt wurden. Hal steht vorzugsweise für Chlor oder Brom.

Die 2-Halogenmethyloxirane der Formel (IV) sind noch nicht bekannt. Sie können jedoch in allgemein üblicher Art und Weise erhalten werden, indem man entweder 2,2-Dihalogenmethyloxirane der Formel (III)

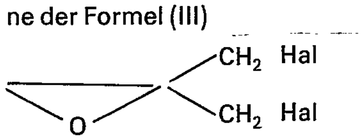

in welcher Hal für Halogen steht, mit einem Nucleophil der Formel (II) umsetzt; oder 3-Halogen-2-halogenmethyl-propene der Formel (VI)

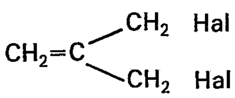

in welcher Hal die oben angegebene Bedeutung hat, zunächst mit einem Nucleophil der Formel (II), gegebenenfalls in Form eines Alkalimetallsalzes, zu Propenen der Formel (VIII)

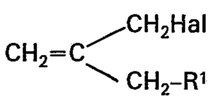 (VIII)

in welcher

Hal und $R^1$ die oben angegebene Bedeutung haben, umsetzt und diese dann in Gegenwart von Persäuren zu den gewünschten 2-Halogenmethyloxiranen der Formel (IV) epoxidiert.

Die ausserdem für das erfindungsgemässe Verfahren (b) als Ausgangsstoffe zu verwendenden Oxirane sind durch die Formel (V) allgemein definiert. In dieser Formel stehen $R^2$ und $R^3$ vorzugsweise für diejenigen Reste, die bereits im Zusammenhang mit der Beschreibung der erfindungsgemässen Stoffe der Formel (I) vorzugsweise für diesen Substituenten genannt wurden.

Die substituierten Oxirane der Formel (V) sind noch nicht bekannt. Sie können jedoch in allgemein üblicher Art und Weise erhalten werden, indem man entweder 2,2-Dihalogenmethyloxirane der Formel (III) mit einem Nucleophil der Formel (II) umsetzt; oder 3-Halogen-2-halogenmethylpropene der Formel (VI) mit einem Nucleophil der Formel (II), gegebenenfalls in Form eines Alkalimetallsalzes, zu Propenen der Formel (IX)

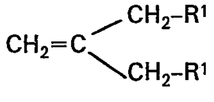 (IX)

in welcher $R^1$ die oben angegebene Bedeutung hat, umsetzt und diese dann in Gegenwart von Persäure zu den gewünschten Oxiranen der Formel (V) epoxidiert; oder Ketone der Formel (X)

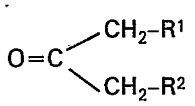 (X)

in welcher

$R^1$ und $R^2$ die oben angegebene Bedeutung haben, in an sich bekannter Art und Weise mit Dimethylsulfoniummethylid epoxidiert.

Als Verdünnungsmittel kommen für die erfindungsgemässen Verfahren (a), (b) vorzugsweise inerte organische Lösungsmittel infrage. Hierzu gehören vorzugsweise Ketone, wie insbesondere Aceton und Methylethylketon; Nitrile, wie insbesondere Acetonitril; Alkohole, wie insbesondere Ethanol und Isopropanol; Ether, wie insbesondere Tetrahydrofuran oder Dioxan; Formamide, wie insbesondere Dimethylformamid; aromatische und halogenierte Kohlenwasserstoffe sowie deren Gemische mit Wasser.

Die erfindungsgmässen Verfahren (a) und (b) werden gegebenenfalls in Gegenwart eines Säurebindemittels vorgenommen. Man kann alle üblicherweise verwendbaren anorganischen oder organischen Säurebinder zugeben, wie Alkalihydroxide und Alkalicarbonate, beispielsweise Natrium- und Kaliumhydroxid sowie Natrium- und Kaliumcarbonat; oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan sowie auch ein entsprechender Überschuss an Imidazol bzw. Triazol.

Die Reaktionstemperaturen können bei der Durchführung der erfindungsgemässen Verfahren (a), (b) in einem grösseren Bereich variiert werden. Im allgemeinen arbeitet man zwischen etwa 20 bis etwa 150°C, vorzugsweise bei 20 bis 120°C.

Bei der Durchführung der erfindungsgemässen Verfahren (a), (b) setzt man jeweils auf 1 Mol der Oxirane der Formeln (III), (IV) und (V) die stöchiometrisch erforderliche bis zum 3-molaren Überschuss erforderliche Menge an Nucleophil der Formel (II) und die stöchiometrischen Menge bis zum 2-molaren Überschuss an Säurebinder ein. Die Isolierung der Verbindungen der Formel (I) erfolgt in üblicher Art und Weise.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Bestimmte Verbindungen der Formel (I) können auch erhalten werden, indem man Di(halogenmethyl)-carbinole der Formel (XI)

$$\begin{array}{c} \qquad\qquad CH_2Hal \\ \qquad\qquad | \\ R^4{-}CH_2{-}C{-}OH \\ \qquad\qquad | \\ \qquad\qquad CH_2Hal \end{array} \qquad (XI)$$

in welcher

Hal die oben angegebene Bedeutung hat und $R^4$ für gegebenenfalls substituiertes Phenylthio, gegebenenfalls substituiertes Phenylamino, gegebenenfalls substituiertes Phenyl-N-alkylamino mit einem Nucleophil der Formel (II) in Gegenwart eines Verdünnungsmittels und in Gegenwart eines Säurebindemittels umsetzt.

Die erfindungsgemässen Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sprosspilze sowie bi-phasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporon-Arten, Epidermaphyton floccosum, Sprosspilze und bi-phasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemässe Wirkstoffen enthalten oder die aus einem oder mehreren erfindungsgemässen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder $\frac{1}{2}$, $\frac{1}{3}$ oder $\frac{1}{4}$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gelee, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gelee können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone,

Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemässen Wirkstoffe sowie von pharmazeutischen Zubereitungen, die einen oder mehrere erfindungsgemässe Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele:

Beispiel 1 und 2

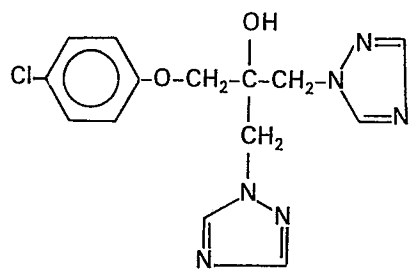

(Beispiel 1)

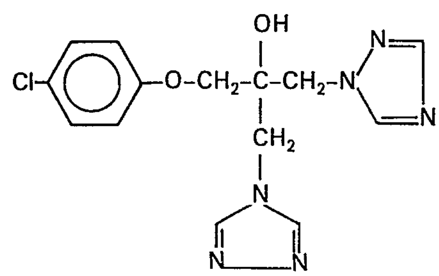

(Beispiel 2)

(Verfahren b)

Zu einem Gemisch von 13,6 g (0,2 Mol) 1,2,4-Triazol und 13,8 g (0,1 Mol) Kaliumcarbonat in 200 ml Aceton werden unter Rühren 9,4 g (0,04 Mol) 2-Chlormethyl-2-(4-chlorphenoxymethyl)-oxiran getropft. Man lässt 15 Stunden bei Raumtemperatur und anschliessend 22 Stunden unter Rückfluss rühren. Das Reaktionsgemisch wird danach kalt filtriert und das Filtrat im Vakuum eingeengt. Der ölige Rückstand wird in Chloroform gelöst, mit Wasser gewaschen, über Natriumsulfat getrocknet und chromatographisch (Kieselgel 60 Merck, Chloroform/Methanol = 20/1) gereinigt. Man erhält 5,8 g (43% der Theorie) 2-(4-Chlorphenoxymethyl)-1,3-di(1,2,4-triazol-1-

yl)-2-hydroxypropan vom Schmelzpunkt 99°C (Beispiel 1) und 2,0 g (15% der Theorie) 2-(4-Chlorphenoxymethyl)-2-hydroxy-1-(1,2,4-triazol-1-yl)-3-(1,2,4-triazol-4-yl)-propan (Beispiel 2) vom Schmelzpunkt 160°C.

Herstellung des Ausgangsproduktes

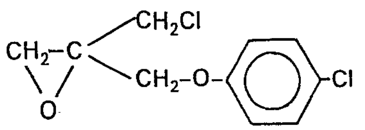

(IV-1)

(1. Variante)

12,85 g (0,1 Mol) 4-Chlorphenol in 50 ml Aceton werden zu einem Gemisch von 14,1 g (0,1 Mol) 2,2-Di(chlormethyl)-oxiran, 13,8 g (0,1 Mol) Kaliumcarbonat in 200 ml Aceton getropft. Man erhitzt 18 Stunden unter Rückfluss, lässt abkühlen und filtriert. Das Filtrat wird im Vakuum eingeengt, der Rückstand in Chloroform gelöst, mit Wasser gewaschen, über Natriumsulfat getrocknet und eingeengt. Nach destillativer Reinigung erhält man 7,6 g (32,5% der Theorie) 2-Chlormethyl-2-(4-chlorphenoxymethyl)-oxiran vom Siedepunkt 150°C/0,5 mbar.

(2. Variante)

0,5 Mol 4-Chlorphenol-natrium in Acetonitril werden zu einer Lösung von 125 g (1 Mol) 3-Chlor-2-chlormethylpropen in 50 ml Acetonitril getropft. Nach Zusatz von 0,5 g Natriumjodid wird das Reaktionsgemisch 12 Stunden unter Rückfluss erhitzt und anschliessend kalt filtriert. Das Filtrat wird im Vakuum eingeengt. Der Rückstand wird in Methylenchlorid gelöst, mit Wasser gewaschen, über Natriumsulfat getrocknet und eingeengt. Man erhält 58,6 g ca. 60%iges 2-Chlormethyl-3-(4-chlorphenoxy)-propen, was in 500 ml Methylenchlorid gelöst und mit 43 g (0,25 Mol) 3-Chlorperbenzoesäure versetzt wird.

Man lässt 24 Stunden bei Raumtemperatur rühren und filtriert. Das Filtrat wird mit wässriger, 10%iger Natriumthiosulfatlösung gewaschen, über Natriumsulfat getrocknet und im Vakuum eingeengt. Nach destillativer Reinigung erhält man 30 g (12,9% der Theorie) 2-Chlormethyl-2-(4-chlorphenoxymethyl)-oxiran vom Brechungsindex $n_D^{20} = 1{,}5465$.

Beispiel 3, 4 und 5

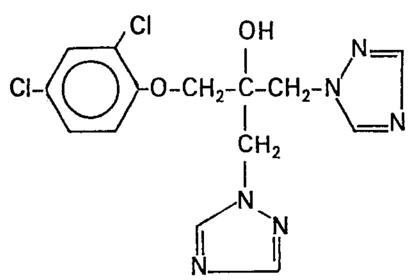

(Beispiel 3)

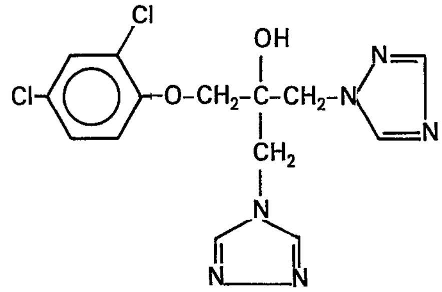

(Beispiel 4)

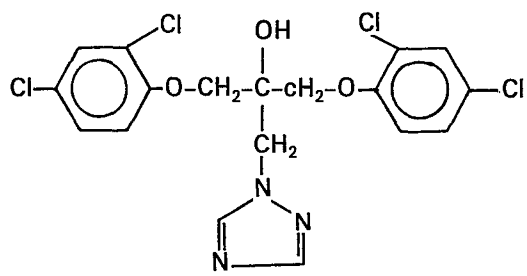

Beispiel 5

(Verfahren b und c)

23,5 g eines Gemisches aus 2-Chlormethyl-2-(2,4-dichlorphenoxymethyl)-oxiran und 2,2-Bis(2,4-dichlorphenoxymethyl)-oxiran werden zu einer Lösung von 20,7 g (0,3 Mol) 1,2,4-Triazol und 13,8 g (0,1 Mol) Kaliumcarbonat in 200 ml Aceton getropft. Man lässt das Reaktionsgemisch 20 Stunden unter Rückfluss rühren. Danach wird kalt filtriert und das Filtrat im Vakuum eingeengt. Der Rückstand wird in Chloroform gelöst, mit Wasser gewaschen, über Natriumsulfat getrocknet und chromatographisch (Kieselgel 60 Merck) gereinigt und getrennt. Zuerst wird mit Chloroform eluiert. Das dabei erhaltene Eluat wird durch Abdestillieren des Lösungsmittels eingeengt und der Rückstand mit Diethylether verrührt. Man erhält 4,8 g 1,3-Bis-(2,4-dichlorphenoxy)-2-hydroxy-2-(1,2,4-triazol-1-yl-methyl)-propan (Beispiel 5) vom Schmelzpunkt 136°C.

Anschliessend wird mit Chloroform/Methanol: 40/1 eluiert. Das dabei erhaltene Eluat wird durch Abdestillieren des Lösungsmittels eingeengt und der Rückstand mit Acetonitril verrührt. Man erhält 3,9 g 2-(2,4-Dichlorphenoxymethyl)-1,3-di(1,2,4-triazol-1-yl)-2-hydroxy-propan (Beispiel 3) vom Schmelzpunkt 138°C.

Zum Schluss wird mit Chloroform/Methanol: 20/1 eluiert. Das dabei erhaltene Eluat wird durch Abdestillieren des Lösungsmittels eingeengt und der Rückstand mit Acetonitril verrührt. Man erhält 3,7 g 2-(2,4-Dichlorphenoxymethyl)-2-hydroxy-1-(1,2,4-triazol-1-yl)-3-(1,2,4-triazol-4-yl)-propan (Beispiel 4) vom Schmelzpunkt 182°C.

Herstellung der Ausgangsprodukte

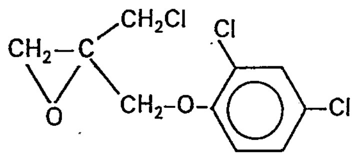

(IV-2)

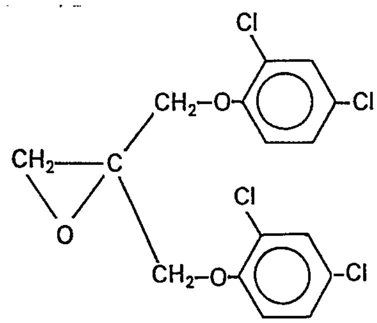

(V-1)

Ein Gemisch von 14,1 g (0,1 Mol) 2,2-Di(chlormethyl)-oxiran, 13,8 g (0,1 Mol) Kaliumcarbonat und 16,3 g (0,1 Mol) 2,4-Dichlorphenol in 150 ml Aceton wird 15 Stunden auf 60°C erhitzt, anschliessend kalt filtriert und das Filtrat im Vakuum eingeengt. Der Rückstand wird in Methylenchlorid aufgenommen, mit Wasser gewaschen, über Natriumsulfat getrocknet und eingeengt. Man erhält 23,5 g eines öligen Gemisches, das aufgrund gaschromatographischer Ermittlung neben 14,9% unumgesetztem 2,2-Di(chlormethyl)-oxiran 47% 2-Chlormethyl-2-(2,4-dichlorphenoxymethyl)-oxiran (Beisp. IV-2) und 24,9% 2,2-Bis(2,4-dichlorphenoxymethyl)-oxiran (Beisp. V-1) enthält.

Dieses Gemisch kann chromatographisch getrennt werden, wobei 2-Chlormethyl-2-(2,4-dichlorphenoxymethyl)-oxiran (Beisp. IV-2) mit einem Brechungsindex $n_D^{20}$= 1,5568 und 2,2-Bis-(2,4-dichlorphenoxymethyl)-oxiran (Beisp. V-1) vom Schmelzpunkt 64°C erhalten werden.

Beispiel 6

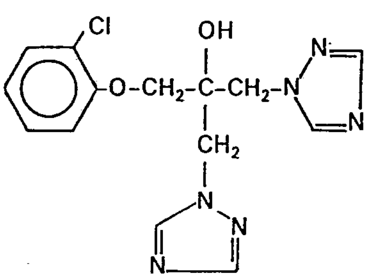

(Verfahren b)

Entsprechend Beispiel 1/2 erhält man aus 8,9 g (0,038 Mol) 2-Chlormethyl-2-(2-chlorphenoxymethyl)-oxiran, 5 g (0,038 Mol) Kaliumcarbonat und 6,9 g (0,1 Mol) 1,2,4-Triazol in 200 ml Aceton nach chromatographischer Reinigung des Reaktionsproduktes (Kieselgel 60 Merck, Chloroform/Methanol = 40/1) 7,1 g (55% der Theorie) 2-(2-Chlorphenoxymethyl)-1,3-di(1,2,4-triazol-1-yl)-2-hydroxy-propan vom Schmelzpunkt 95°C.

Herstellung des Ausgangsproduktes

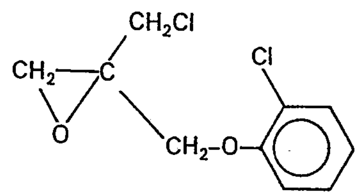

(IV-3)

Entsprechend Beispiel 1/2 (Herstellung des Ausgangsproduktes, 1. Variante) erhält man aus 12,85 g (0,1 Mol) 2-Chlorphenol in 50 ml Aceton und 14,1 g (0,1 Mol) 2,2-Di(chlormethyl)-oxiran sowie 13,8 g (0,1 Mol) Kaliumcarbonat in 200 ml Aceton 9,1 g (39% der Theorie) 2-Chlormethyl-2-(2-chlorphenoxymethyl)-oxiran vom Brechungsindex $n_D^{20}$= 1,5463.

Beispiel 7 und 8

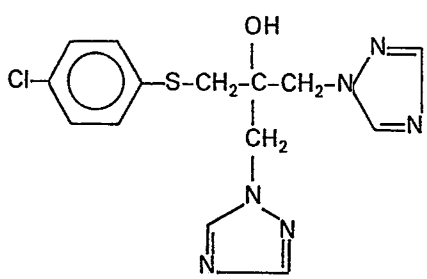

(Beispiel 7)

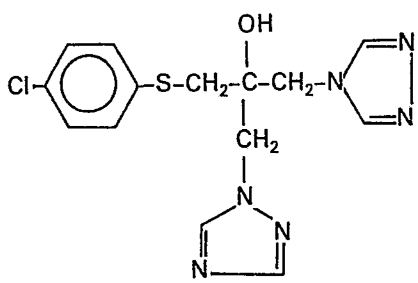

(Beispiel 8)

(Verfahren b)

Entsprechend Beispiel 1/2 erhält man aus 14,8 g (0,06 Mol) 2-Chlormethyl-2-(4-chlorphenylthiomethyl)-oxiran, 8,3 g (0,12 Mol) 1,2,4-Triazol und 8,3 g (0,06 Mol) Kaliumcarbonat in 200 ml Aceton, 12,1 g (57,5% der Theorie) 2-(4-Chlorphenylthiomethyl)-1,3-di-(1,2,4-triazol-1-yl)-2-hydroxy-propan (Beispiel 7) vom Schmelzpunkt 150°C und 4,6 g (22% der Theorie) 2-(4-Chlorphenylthiomethyl)-2-hydroxy-1-(1,2,4-triazol-1-yl)-3-(1,2,4-triazol-4-yl)-propan (Beispiel 8) vom Schmelzpunkt 186°C.

Herstellung des Ausgangsproduktes

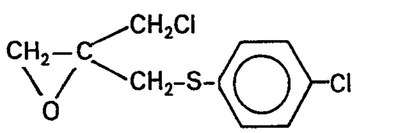 (IV-4)

5,4 (0,1 Mol) Natriummethylat werden portionsweise in ein Gemisch von 16,9 g (0,12 Mol) 2,2-Di(chlormethyl)-oxiran und 14,5 g (0,1 Mol) 4-Chlorthiophenol in 200 ml Acetonitril eingetragen. Man lässt das Reaktionsgemisch 4 Stunden nachrühren, filtriert und engt das Filtrat im Vakuum ein. Der Rückstand wird in Methylenchlorid gelöst, in Wasser gewaschen, über Natriumsulfat getrocknet und eingeengt. Das verbleibende Öl wird destilliert. Man erhält 18,2 g (73% der Theorie) 2-Chlormethyl-2-(4-chlorphenylthiomethyl)-oxiran vom Brechungsindex $n_D^{20}$= 1,5895.

In entsprechender Weise und gemäss den erfindungsgemässen Verfahren werden die folgenden Endprodukte der allgemeinen Formel (I)

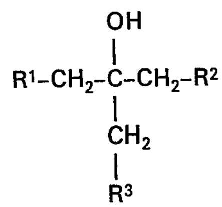 (I)

in Tabelle 1 erhalten:

Tabelle 1

| Beispiel Nr. | $R^1$ | $R^2$ | $R^3$ | Schmp. (°C) oder $n_D^{20}$ |
|---|---|---|---|---|
| 9 | 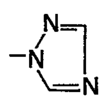 | 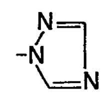 | 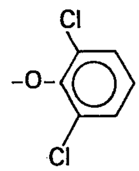 | 166 |
| 10 | 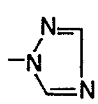 | 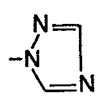 | 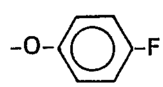 | 90 |
| 11 | 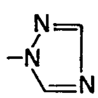 | 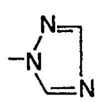 | 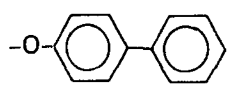 | 140 |
| 12 | 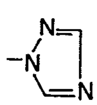 | 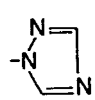 | 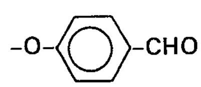 | 179 |
| 13 | 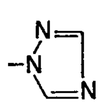 | 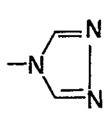 | 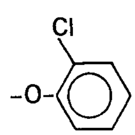 | 146 |
| 14 | 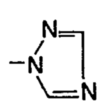 | 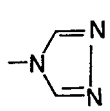 | 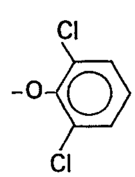 | 150 |
| 15 | 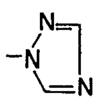 | 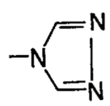 | 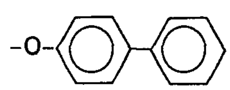 | 204 |

Tabelle 1 (Fortsetzung)

| Beispiel Nr. | R¹ | R² | R³ | Schmp. (°C) oder $n_D^{20}$ |
|---|---|---|---|---|
| 16 | 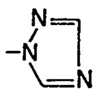 | 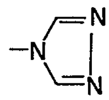 | 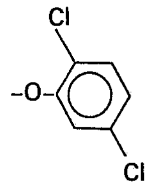 | 166 |
| 17 | 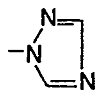 | 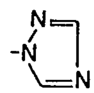 | 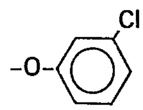 | 92 |
| 18 | 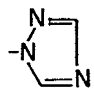 | 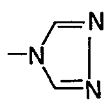 | 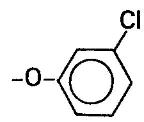 | 130 |
| 19 | 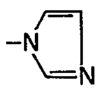 | 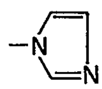 | 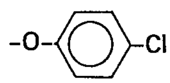 | 100 |
| 20 | 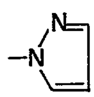 | 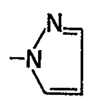 | 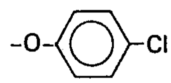 | 72 |
| 21 | 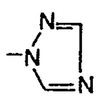 | 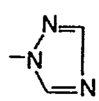 | 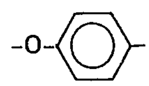 | 84 |
| 22 | 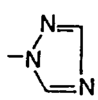 | 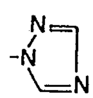 | 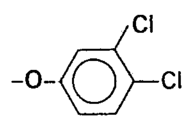 | 158 |
| 23 | 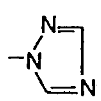 | 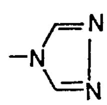 | 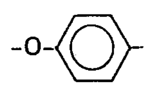 | 112 |
| 24 | 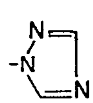 | 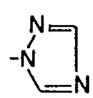 | 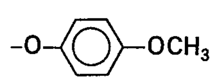 | 118 |
| 25 | 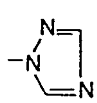 | 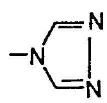 | 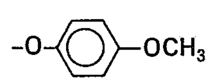 | 162 |
| 26 | 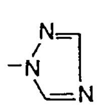 | 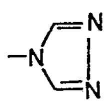 | 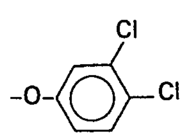 | 188 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 27 | 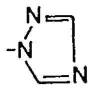 | 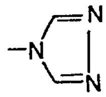 | 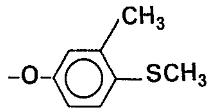 | 70 |
| 28 | 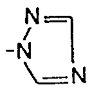 | 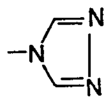 | 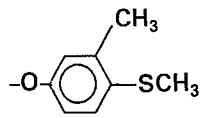 | 138 |
| 29 | 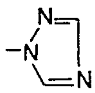 | 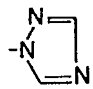 | 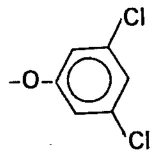 | 162 |
| 30 | 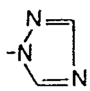 | 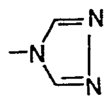 | 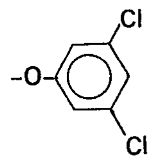 | 174 |
| 31 | 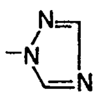 | 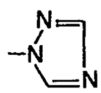 | 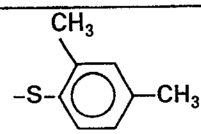 | 134 |
| 32 | 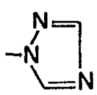 | 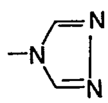 | 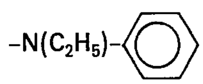 | 84 |
| 33 | 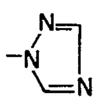 | 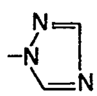 | 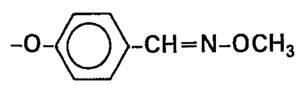 | 120 |
| 34 | 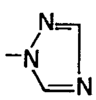 | 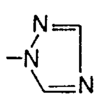 | 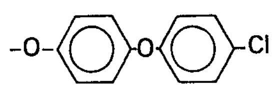 | 74 |
| 35 | 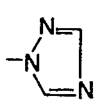 | 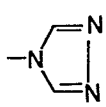 | 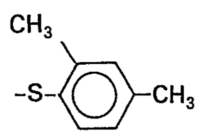 | 95 |
| 36 | 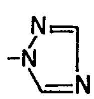 | 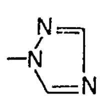 | 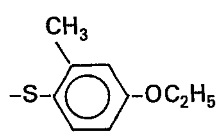 | 75 |
| 37 | 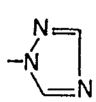 | 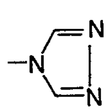 | 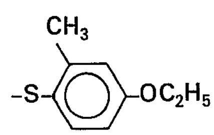 | 93 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 39 | 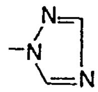 | 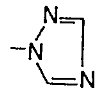 | 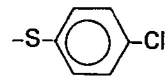 | 75 |
| 40 | 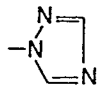 | 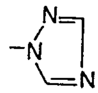 | 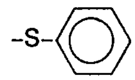 | 70 |
| 41 | 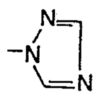 | 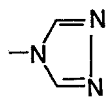 | 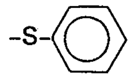 | 90 |
| 42 | 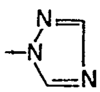 | 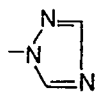 | 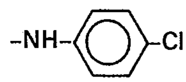 | 116 |
| 43 | 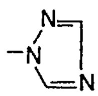 | 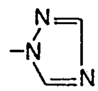 | 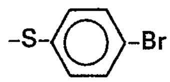 | 142 |
| 44 | 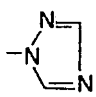 | 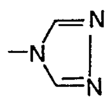 | 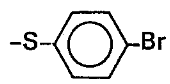 | 186 |
| 45 | 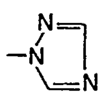 | 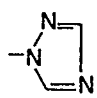 | 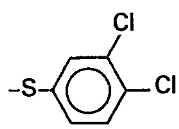 | 149 |
| 46 | 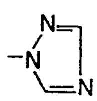 | 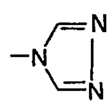 | 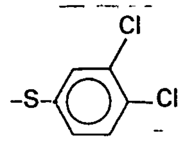 | 155 |
| 47 | 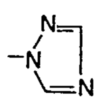 | 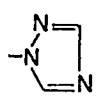 | 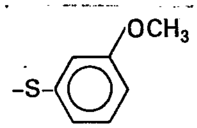 | 1,582 |
| 48 | 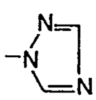 | 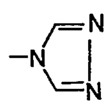 | 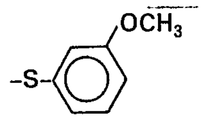 | 45 |
| 49 | 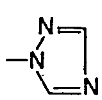 | 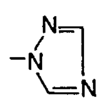 | 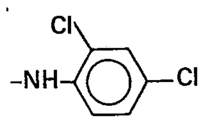 | 130 |
| 50 | 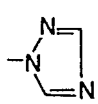 | 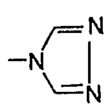 | 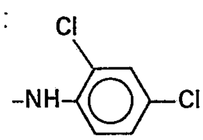 | 150 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 51 | 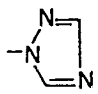 | 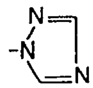 | 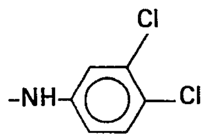 | 126 |
| 52 | 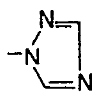 | 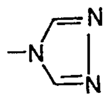 | 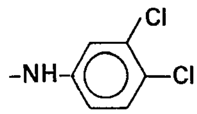 | 95 |
| 53 | 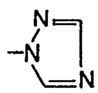 | 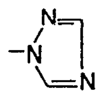 | 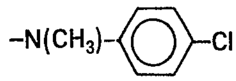 | 142 |
| 54 | 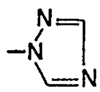 | 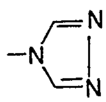 | 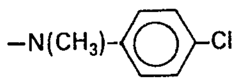 | 177 |
| 55 | 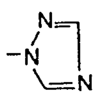 | 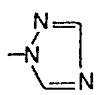 | 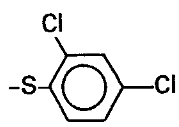 | 90 |
| 56 | 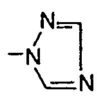 | 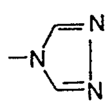 | 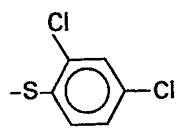 | 70 |
| 57 | 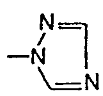 | 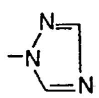 | 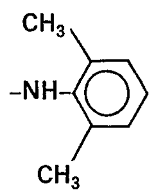 | 1,5620 |
| 58 | 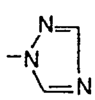 | 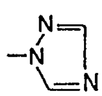 | 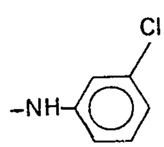 | 1,5698 |
| 59 | 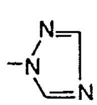 | 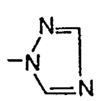 | 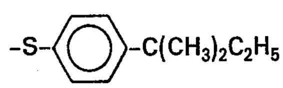 | 1,5600 |
| 60 | 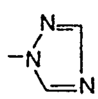 | 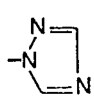 | 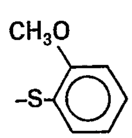 | 80 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 61 | 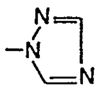 | 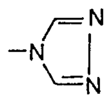 | 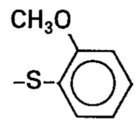 | 108 |
| 62 | 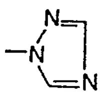 | 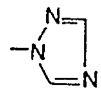 | 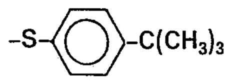 | 1,5608 |
| 63 | 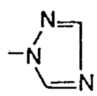 | 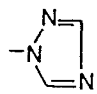 | 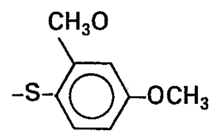 | 126 |
| 64 | 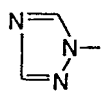 | 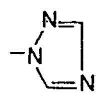 | 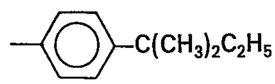 | 142 |
| 65 | 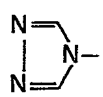 | 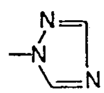 | 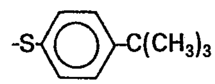 | 144 |
| 66 | 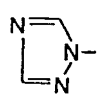 | 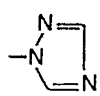 | 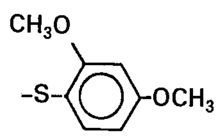 | zähes Öl |
| 67 | 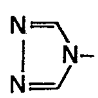 | 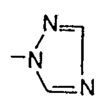 | 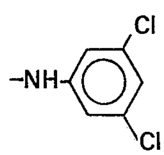 | 168 |
| 68 | 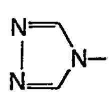 | 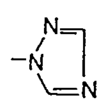 | 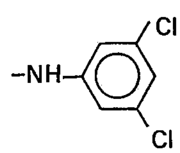 | 108 |
| 69 | 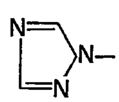 | 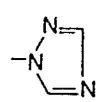 | 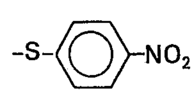 | 150 |
| 70 | 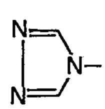 | 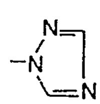 | 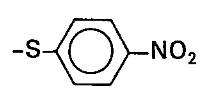 | 188 |
| 71 | 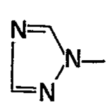 | 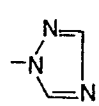 | 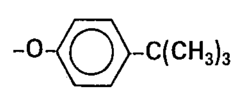 | 98 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 72 | 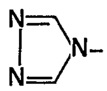 | 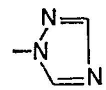 | 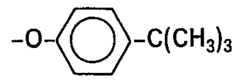 | 164 |
| 73 | 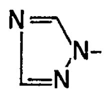 | 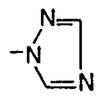 | 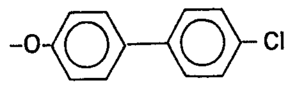 | 166 |
| 74 | 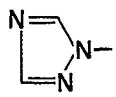 | 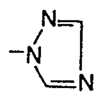 | 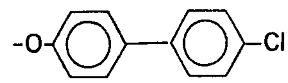 | 194 |
| 75 | 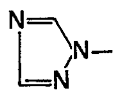 | 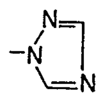 | 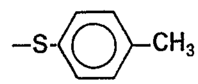 | 128 |
| 76 | 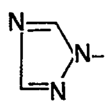 | 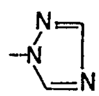 | 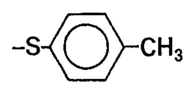 | 141 |
| 77 | 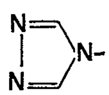 | 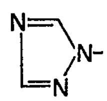 | 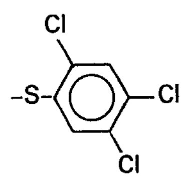 | 115 |
| 78 | 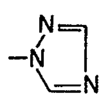 | 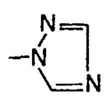 | 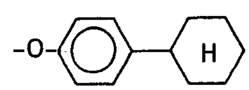 | 96 |
| 79 | 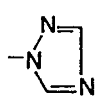 | 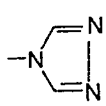 | 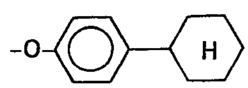 | 159 |
| 80 | 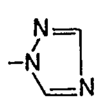 | 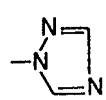 | 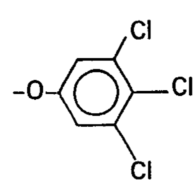 | 184 |
| 81 | 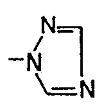 | 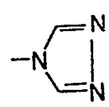 | 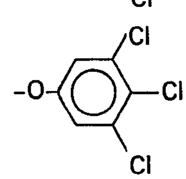 | 186 |
| 82 | 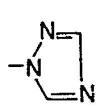 | 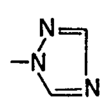 | 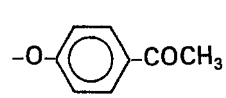 | 142 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 83 | 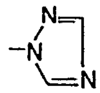 | 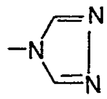 | 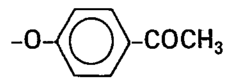 | 190 |
| 84 | 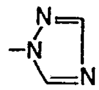 | 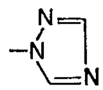 | 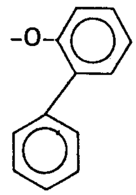 | 120 |
| 85 | 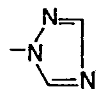 | 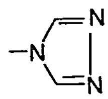 | 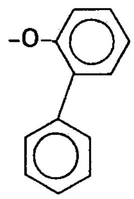 | 130 |
| 86 | 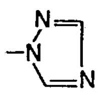 | 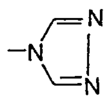 | 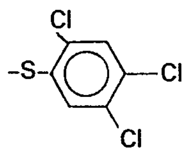 | 80 |
| 87 | 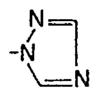 | 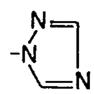 | 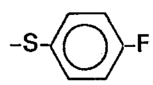 | 114 |
| 88 | 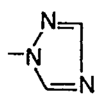 | 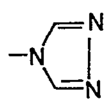 | 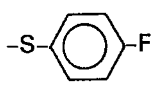 | 169 |
| 89 | 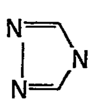 | 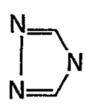 | 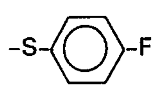 | 140 |
| | | | 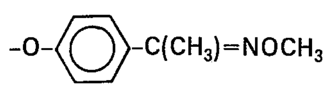 | 154 |
| 90 | 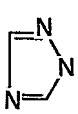 | 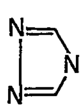 | 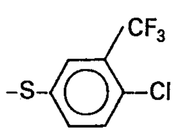 | 50 |
| | | | 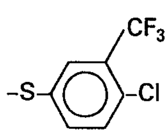 | 75 |

Tabelle 1 (Fortsetzung)

| Beispiel Nr. | $R^1$ | $R^2$ | $R^3$ | Schmp. (°C) oder $n_D^{20}$ |
|---|---|---|---|---|
| 94 | 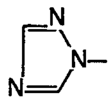 | 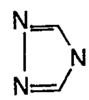 | 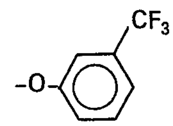 | 97 |
| 95 | 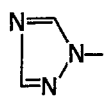 | 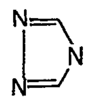 | 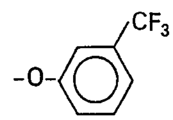 | 110 |
| 96 | 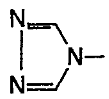 | 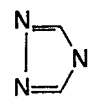 | | |
| 97 | 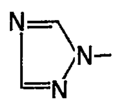 | 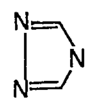 | 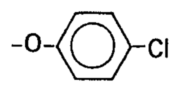 | 100 |
| 98 | 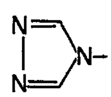 | 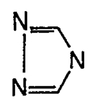 | | |
| 99 | 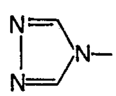 | 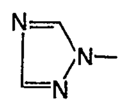 | 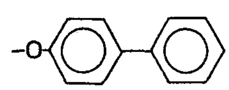 | |

Entsprechend den Herstellungsbeispielen und den allgemeinen Verfahrensangaben werden die folgenden Zwischenprodukte der allgemeinen Formel (IV)

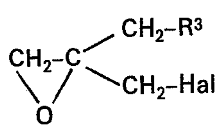

(IV)

in Tabelle 2 erhalten:

Tabelle 2

| $R^3$ | Hal | Siedepunkt (°C)/ mbar bzw. $n_D^{20}$ |
|---|---|---|
| 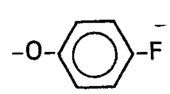 | Cl | 100/0,2 |
| 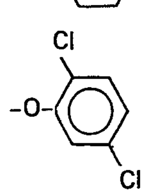 | Cl | 145/0,1 |

Tabelle 2 (Fortsetzung)

| $R^3$ | Hal | Siedepunkt (°C)/ mbar bzw. $n_D^{20}$ |
|---|---|---|
| 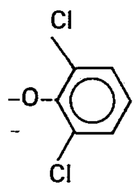 | Cl | 155/0,3 |
| 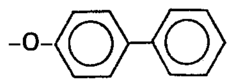 | Cl | 1,5842 |
| 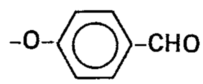 | Cl | 190/0,2 |
| 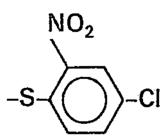 | Cl | 1,6270 |
| 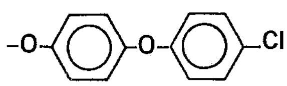 | Cl | 1,5442 |
| 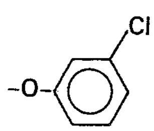 | Cl | 140/0,5 |
| 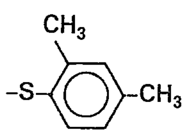 | Cl | 122/0,1 |
| 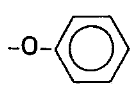 | Cl | 110/0,1 |
| 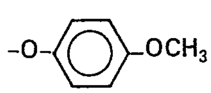 | Cl | 120/0,2 |
| 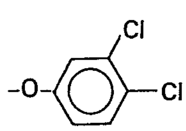 | Cl | 140/0,2 |
| 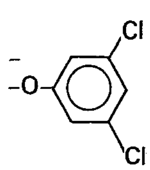 | Cl | 140/0,2 |

Tabelle 2 (Fortsetzung)

| $R^3$ | Hal | Siedepunkt (°C)/ mbar bzw. $n_D^{20}$ |
|---|---|---|
| 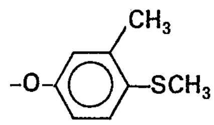 | Cl | 160/0,2 |
| 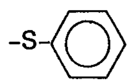 | Cl | 120/0,3 |
| 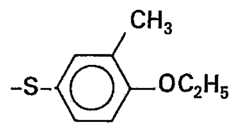 | Cl | 138/0,1 |
| 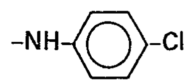 | Cl | 1,5843 |
| 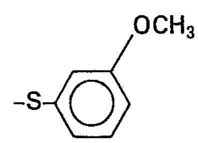 | Cl | 160/0,3 |
| 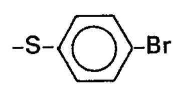 | Cl | 158/0,3 |
| 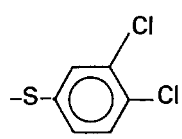 | Cl | 155/0,3 |

Entsprechend den Herstellungsbeispielen und den allgemeinen Verfahrensangaben werden die folgenden Zwischenprodukte der allgemeinen Formel (V)

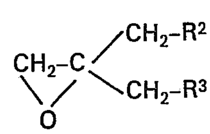 (V)

in Tabelle 3 erhalten:

Tabelle 3

| $R^3$ | $R^2$ | Schmelzpunkt (°C) bzw. $n_D^{20}$ |
|---|---|---|
| 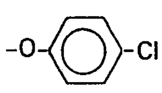 | 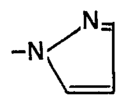 | 1,5590 |

Die Herstellung der gegebenenfalls als Vorprodukte zu verwendenden Propen-Derivate der Formeln (VII) und (IX) sei anhand der folgenden Beispiele erläutert:

Beispiel α

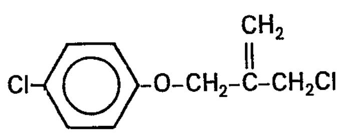

(Beispiel VIII-1)

Eine Lösung von 64,25 g (0,5 Mol) 4-Chlorphenol in 100 ml Aceton wird bei 60°C zu einem Gemisch von 125 g (1 Mol) 3-Chlor-2-chlormethyl-propen und 34,5 g (0,25 Mol) Kaliumcarbonat in 500 ml Aceton getropft. Man lässt 18 Stunden bei dieser Temperatur nachrühren, filtriert und engt das Filtrat ein. Der ölige Rückstand wird in Methylenchlorid gelöst, einmal mit 200 ml 10%iger Natronlauge und danach zweimal mit je 500 ml Wasser gewaschen, über Natriumsulfat getrocknet und eingeengt. Der ölige Rückstand wird destilliert. Man erhält 43,2 g (41,5% der Theorie) 2-Chlormethyl-3-(4-chlorphenoxy)-propen (Beispiel VIII-1) vom Siedepunkt 80 bis 90°C/0,05 mbar.

Beispiel β

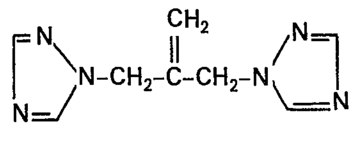

(Beispiel VIII-2)

Ein Gemisch von 25 g (0,2 Mol) 3-Chlor-2-chlormethyl-propen, 27,6 g (0,4 Mol) 1,2,4-Triazol und 27,6 (0,2 Mol) Kaliumcarbonat in 200 ml Aceton wird 15 Stunden unter Rückfluss erhitzt. Nach dem Abkühlen wird filtriert und das Filtrat in Vakuum eingeengt. Der ölige Rückstand wird chromatographiert (Kieselgel 60 Merck, Chloroform). Man erhält 23 g (63% der Theorie) 3-(1,2,4-Triazol-1-yl)-2-(1,2,4-triazol-1-yl-methyl)-propen vom Schmelzpunkt 52°C.

Verwendungsbeispiele

In dem nachfolgenden Beispiel werden die nachstehend angegebenen Verbindungen als Vergleichssubstanzen eingesetzt:

A)

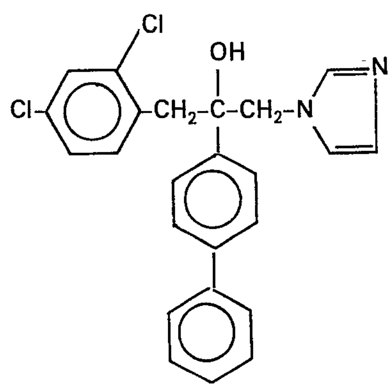

bekannt aus DE-OS 2 820 489

B)

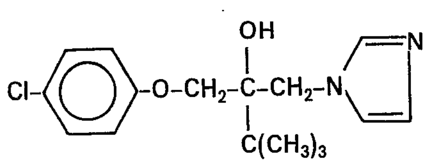

bekannt aus DE-OS 3 018 865

C)

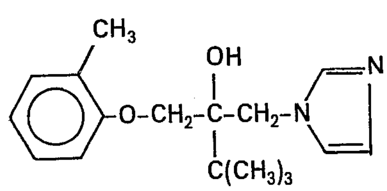

bekannt aus DE-OS 3 018 865

D) 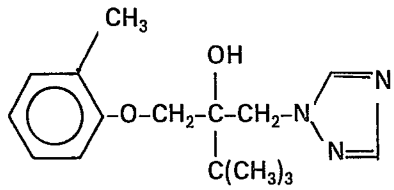

bekannt aus DE-OS 3 018 865

E) 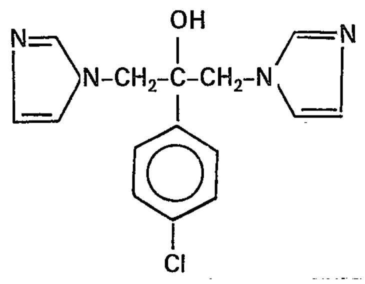

bekannt aus EP-OS 44 605

Beispiel A

Antimykotische in-vivo-Wirksamkeit (oral) bei Mäusecandidose

Versuchsbeschreibung:

Mäuse vom Typ SPF-$CF_1$ wurden intravenös mit 1–2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50–100 mg/kg Körpergewicht der Präparate oral behandelt.

Ergebnis:

Unbehandelte Tiere starben 3 bis 6 Tage post infectionem. Die Überlebensrate am 6. Tag post infectionem betrug bei unbehandelten Kontrolltieren etwa 5%.

In diesem Test zeigten z.B. die erfindungsgemässen Verbindungen 1, 3, 4, 7, 10 und 11 eine bessere Wirkung als die aus dem Stand der Technik bekannten Verbindungen (A), (B), (C), (D) und (E).

Zeichenerklärung:

| | |
|---|---|
| +++++ = sehr gute Wirkung | = 90% Überlebende am 6. Tag p.i. |
| ++++ = gute Wirkung | = 80% Überlebende am 6. Tag p.i. |
| +++ = Wirkung | = 60% Überlebende am 6. Tag p.i. |
| ++ = schwache Wirkung | = 40% Überlebende am 6. Tag p.i. |
| + = Spur Wirkung | = <40% Überlebende am 6. Tag p.i. |

k.W. = keine Wirkung

Tabelle A

Antimykotische in-vivo-Wirksamkeit (oral) bei Mäusecandidose

| Wirkstoff | Wirkung |
|---|---|
| (A) (bekannt) | k.W. |
| (B) (bekannt) | k.W. |
| (C) (bekannt) | k.W. |
| (D) (bekannt) | k.W. |
| (E) (bekannt) | k.W. |

Verbindungen gemäss den Herstellungs-Beispielen:

| | |
|---|---|
| 1 | +++++ |
| 3 | +++++ |
| 4 | +++ |
| 7 | +++++ |
| 10 | +++++ |
| 11 | +++++ |

Beispiel 8 / Formulierungen

1. Lösung:

| | |
|---|---|
| Wirkstoff gemäss Formel (I) | 10 g |
| Alkohol, rein (96%ig) | 300 g |
| Isopropylmyristat | 526 g |
| | 836 g |

2. Creme:

| | |
|---|---|
| Wirkstoff gemäss Formel (I) | 10 g |
| Arlacel 60 | 20 g |
| (Sorbitan-monostearat) Tween 60 | 15 g |
| Polyoxyethylen(2)-sorbitan-monostearat | |
| Walrat, künstlich | 30 g |
| (Mischung von Estern von gesättigten Fettsäuren $C_{14}$–$C_{18}$ und Fettalkoholen $C_{14}$–$C_{18}$) | |
| Lanette O | 100 g |
| (Gemisch aus Cetyl-Alkohol und Stearyl-Alkohol) | |
| Entanol G | 135 g |
| (2-Octyl-dodecanol) | |
| Benzylalkohol | 10 g |
| Wasser, entmineralisiert | 680 g |
| | 1000 g |

**Patentansprüche**

1. Substituierte tert.-Butanolderivate der allgemeinen Formel (I)

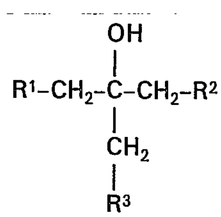 (I)

in welcher

$R^1$ und $R^2$ gleich oder verschieden sind und für

Imidazol-1-yl, 1,2,4-Triazol-1-yl; 1,2,4-Triazol-4-yl oder Pyrazol-1-yl stehen und

$R^3$ für jeweils gegebenenfalls einfach oder mehrfach, gleich oder verschieden substituiertes Phenoxy, Phenylthio, Phenylamino oder Phenyl-N-alkyl-amino mit 1 bis 4 Kohlenstoffatomen im Alkylteil steht, wobei als Phenylsubstituenten jeweils infrage kommen: Halogen, Alkyl mit 1 bis 4 Kohlenstoffatomen, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen, Cycloalkyl mit 5 oder 6 Kohlenstoffatomen, Nitro, Cyano, Hydroxycarbonyl, Alkylcarbonyl oder Alkoxycarbonyl mit jeweils 1 bis 4 Kohlenstoffatomen im Alkylteil, gegebenenfalls einfach oder mehrfach, gleich oder verschieden durch Halogen, Nitro, Trifluormethyl und Alkyl, mit 1 bis 2 Kohlenstoffatomen substituiertes Phenyl oder Phenoxy, die Aldehydgruppe sowie der Oxim- bzw. Oximether-Rest, und deren physiologisch verträgliche Säureadditionssalze.

2. Substituierte tert.-Butanolderivate der allgemeinen Formel (I)

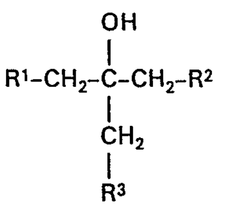

(I)

in welcher

$R^1$ und $R^2$ gleich oder verschieden sind und für Imidazol-1-yl; 1,2,4-Triazol-1-yl; 1,2,4-Triazol-4-yl oder Pyrazol-1-yl stehen und

$R^3$ für jeweils gegebenenfalls einfach bis dreifach, gleich oder verschieden substituiertes Phenoxy, Phenylthio, Phenylamino oder Phenyl-N-alkyl-amino mit 1 bis 2 Kohlenstoffatomen im Alkylteil steht, wobei als Phenylsubstituenten jeweils infrage kommen: Fluor, Chlor, Brom, Methyl, Ethyl, Isopropyl, tert.-Butyl, 2-Methyl-but-2-yl, Methoxy, Ethoxy, Methylthio, Trifluormethyl, Trifluormethoxy, Trifluormethylthio, Cyclohexyl, Nitro, Cyano, Hydroxycarbonyl, Methylcarbonyl, Methoxycarbonyl, Ethoxycarbonyl, jeweils gegebenenfalls einfach bis dreifach, gleich oder verschieden durch Chlor, Nitro, Trifluormethyl oder Methyl substituiertes Phenyl und Phenoxy, die Aldehydgruppe, Hydroxyiminoethyl und Methoxyiminomethyl.

3. 2-(4-Chlorphenoxymethyl)-1,3-di-(1,2,4-triazol-1-yl)-2-hydroxypropan.

4. 2-(2,4-Dichlorphenoxymethyl)-1,3-di(1,2,4-triazol-1-yl)-2-hydroxypropan.

5. 2-(2,4-Dichlorphenoxymethyl)-2-hydroxy-1-(1,2,4-triazol-1-yl)-3-(1,2,4-triazol-4-yl)-propan.

6. 2-(4-Chlorphenylthiomethyl)-1,3-di-(1,2,4-triazol-1-yl)-2-hydroxypropan.

7. 1,2-Di-(1,2,4-triazol-1-yl)-3-(4-fluor-phenoxy)-2-hydroxypropan.

8. 1,2-Di-(1,2,4-triazol-1-yl)-3-(4-phenylphenoxy)-2-hydroxypropan.

9. Substituierte tert.-Butanolderivate gemäss Anspruch 1 zur Bekämpfung von Mykosen.

10. Substituierte tert.-Butanolderivate gemäss Ansprüchen 2 bis 8 zur Bekämpfung von Mykosen.

11. Verfahren zur Herstellung von substituierten tert.-Butanolderivaten der allgemeinen Formel

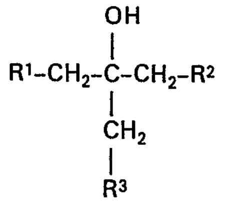

(I)

in welcher

$R^1$ und $R^2$ gleich oder verschieden sind und für Imidazol-1-yl, 1,2,4-Triazol-1-yl; 1,2,4-Triazol-4-yl oder Pyrazol-1-yl stehen und

$R^3$ für jeweils gegebenenfalls einfach oder mehrfach, gleich oder verschieden substituiertes Phenoxy, Phenylthio, Phenylamino oder Phenyl-N-alkyl-amino mit 1 bis 4 Kohlenstoffatomen im Alkylteil steht, wobei als Phenylsubstituenten jeweils infrage kommen: Halogen, Alkyl mit 1 bis 4 Kohlenstoffatomen, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen, Cycloalkyl mit 5 oder 6 Kohlenstoffatomen, Nitro, Cyano, Hydroxycarbonyl, Alkylcarbonyl oder Alkoxycarbonyl mit jeweils 1 bis 4 Kohlenstoffatomen im Alkylteil, gegebenenfalls einfach oder mehrfach, gleich oder verschieden durch Halogen, Nitro, Trifluormethyl und Alkyl, mit 1 bis 2 Kohlenstoffatomen substituiertes Phenyl oder Phenoxy, die Aldehydgruppe sowie dem Oxim- bzw. Oximether-Rest und/oder deren physiologisch verträglichen Säureadditions-Salzen, dadurch gekennzeichnet, dass man ein Nucleophil der Formel (II)

$$R^1\text{-H} \qquad \text{(II)}$$

in welcher

$R^1$ die oben angegebene Bedeutung hat,

a) mit 2-Halogenmethyloxiranen der Formel (IV)

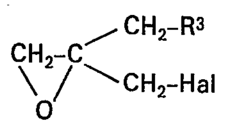

(IV)

in welcher

$R^3$ die oben angegebene Bedeutung hat, und Hal für Halogen steht, in Gegenwart eines Verdünnungsmittels und in Gegenwart eines Säurebindemittels umsetzt, oder

b) mit substituierten Oxiranen der Formel (V)

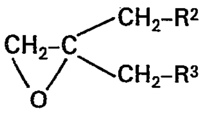

(V)

in welcher

$R^2$ und $R^3$ die oben angegebene Bedeutung haben,

in Gegenwart eines Verdünnungsmittels und in Gegenwart eines Säurebindemittels umsetzt.

12. Arzneimittel, gekennzeichnet durch einen Gehalt an mindestens einem substituierten tert.-Butanolderivat der Formel (I) in Anspruch 1.

13. Verfahren zur Herstellung von antimykotischen Mitteln, dadurch gekennzeichnet, dass man substituierte tert.-Butanolderivate gemäss Anspruch 1 mit inerten, nicht-toxischen, pharmazeutisch geeigneten Trägerstoffen vermischt.

14. Substituierte tert.-Butanolderivate gemäss Anspruch 1 zur Verwendung bei der Bekämpfung von Mykosen.

**Revendications**

1. Dérivés substitués du tertio-butanol, de formule générale (I):

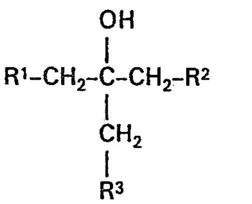

(I)

dans laquelle:

$R^1$ et $R^2$ sont identiques ou différents et représentent un groupe imidazole-1-yle, 1,2,4-triazole-1-yle; 1,2,4-triazole-4-yle ou pyrazole-1-yle, et

$R^3$ représente un groupe phénoxy, phénylthio, phénylamino ou phényl-N-alkylamino comportant 1 à 4 atomes de carbone dans la partie alkyle (éventuellement substitués chacun, une ou plusieurs fois, de façon identique ou différente), et l'on peut chaque fois citer comme substituants du noyau phényle: un atome d'halogène, un groupe alkyle ayant 1 à 4 atomes de carbone, alcoxy et alkylthio ayant chacun 1 à 4 atomes de carbone; un groupe halogénoalkyle, halogénoalcoxy et halogénoalkylthio ayant chacun 1 à 4 atomes de carbone et 1 à 5 atomes d'halogène identiques ou différents, comme des atomes de fluor ou de chlore, un groupe cycloalkyle ayant 5 ou 6 atomes de carbone, un groupe nitro, cyano, hydroxycarbonyle, alkylcarbonyle ou alcoxycarbonyle ayant chacun 1 à 4 atomes de carbone dans la partie alkyle, un groupe phényle ou phénoxy (éventuellement substitués une ou plusieurs fois, de façon identique ou différente, par un atome d'halogène, un groupe nitro, un groupe trifluorométhyle et alkyle ayant 1 à 2 atomes de carbone), le groupe aldéhyde ainsi que le reste oxime ou éther d'oxime, et leurs sels d'addition d'acides physiologiquement tolérables.

2. Dérivés substitués du tertio-butanol, de formule générale (I):

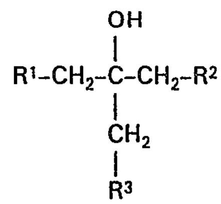

(I)

dans laquelle:

$R^1$ et $R^2$ sont identiques ou différents et représentent un groupe imidazole-1-yle; 1,2,4-triazole-1-yle; 1,2,4-triazole-4-yle ou pyrazole-1-yle, et

$R^3$ représente un groupe phénoxy, phénylthio, phénylamino ou phényl-N-alkylamino ayant 1 à 2 atomes de carbone dans la partie alkyle (éventuellement substitués chacun, une ou plusieurs fois, de façon identique ou différente) et l'on peut citer comme substituants du noyau phényle, à chaque fois: un atome de fluor, de chlore, de brome, un groupe méthyle, éthyle, isopropyle, tertio-butyle, 2-méthyl-but-2-yle, méthoxy, éthoxy, méthylthio, trifluorométhyle, trifluorométhoxy, trifluorométhylthio, cyclohexyle, nitro, cyano, hydroxycarbonyle, méthylcarbonyle, méthoxycarbonyle, éthoxycarbonyle, un groupe phényle et phénoxy (chaque fois éventuellement substitués, une à trois fois, de façon identique ou différente, par du chlore, par un groupe nitro, trifluorométhyle ou méthyle), le groupe aldéhyde, le groupe hydroxyiminoéthyle et méthoxyiminométhyle.

3. Le 2-(4-chlorophénoxyméthyl)-1,3-di-(1,2,4-triazole-1-yl)-2-hydroxypropane.

4. Le 2-(2,4-dichlorophénoxyméthyl)-1,3-di (1,2,4-triazole-1-yl)-2-hydroxypropane.

5. Le 2-(2,4-dichlorophénoxyméthyl)-2-hydroxy-1-(1,2,4-triazole-1-yl)-3-(1,2,4-triazole-4-yl)-propane.

6. Le 2-(4-chlorophénylthiométhyl)-1,3-di-(1,2,4-triazole-1-yl)-2-hydroxypropane.

7. Le 1,2-di-(1,2,4-triazole-1-yl)-3-(4-fluorophénoxy)-2-hydroxypropane.

8. Le 1,2-di-(1,2,4-triazole-1-yl)-3-(4-phénylphénoxy)-2-hydroxypropane.

9. Dérivés substitués du tertio-butanol selon la revendication 1, pour combattre des mycoses.

10. Dérivés substitués du tertio-butanol selon les revendications 2 à 8 pour combattre les mycoses.

11. Procédé pour préparer des dérivés substitués du tertio-butanol de formule générale:

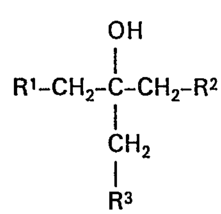

(I)

dans laquelle:

$R^1$ et $R^2$ sont identiques ou différents et représentent un groupe imidazole-1-yl, 1,2,4-triazole-1-yle; 1,2,4-triazole-4-yle ou pyrazole-1-yle, et

$R^3$ représente un groupe phénoxy, phénylthio, phénylamino ou phényl-N-alkylamino comportant 1 à 4 atomes de carbone dans la partie alkyle (éventuellement substitués chacun, une ou plusieurs fois, de façon identique ou différente), et l'on peut chaque fois citer comme substituants du noyau phényle: un atome d'halogène, un groupe alkyle ayant 1 à 4 atomes de carbone, alcoxy et alkylthio ayant chacun 1 à 4 atomes de carbone; un groupe halogénoalkyle, halogénoalcoxy et halogénoalkylthio ayant chacun 1 à 4 atomes de carbone et 1 à 5 atomes d'halogène identiques ou différents, comme des atomes de fluor ou de chlore, un groupe cycloalkyle ayant 5 ou 6 atomes de carbone, un groupe nitro, cyano, hydroxycarbonyle, alkylcarbonyle ou alcoxycarbonyle ayant chacun 1 à 4 atomes de carbone dans la partie alkyle, un groupe phényle ou phénoxy (éventuellement substitués une ou plusieurs fois, de façon identique ou différente, par un atome d'halogène, un groupe nitro, un groupe trifluorométhyle et alkyle ayant 1 à 2 atomes de carbone), le groupe aldéhyde ainsi que le reste oxime ou éther d'oxime, et/ou leurs sels d'addition d'acides physiologiquement compatibles, procédé caractérisé en ce qu'on fait réagir un nucléophile de formule (II):

$$R^1\text{-}H \qquad (II)$$

dans laquelle $R^1$ a le sens indiqué ci-dessus,

a) avec des 2-halogénométhyloxirannes de formule (IV)

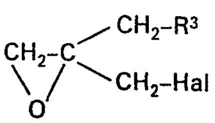

(IV)

dans laquelle:

$R^3$ a le sens indiqué ci-dessus et Hal représente un halogène, en présence d'un diluant et en présence d'un agent de fixation des acides, ou

b) avec des oxirannes substitués de formule (V)

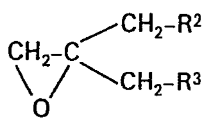

(V)

dans laquelle:

$R^2$ et $R^3$ ont le sens indiqué ci-dessus, en présence d'un diluant et en présence d'un agent de fixation des acides.

12. Médicament, caractérisé en ce qu'il contient au moins un dérivé substitué de tertio-butanol de formule (I) de la revendication 1.

13. Procédé pour préparer des produits antifongiques, caractérisé en ce qu'on mélange des dérivés substitués du tertio-butanol, selon la revendication 1, avec des véhicules ou supports pharmaceutiquement convenables, inertes, non toxiques.

14. Dérivés substitués du tertio-butanol selon la revendication 1, destinés à servir à combattre des mycoses.

**Claims**

1. Substituted tert.-butanol derivatives of the general formula (I)

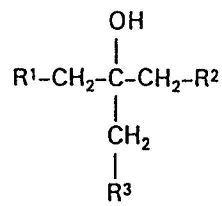

(I)

in which

$R^1$ and $R^2$ are identical or different and represent imidazol-1-yl, 1,2,4-triazol-1-yl; 1,2,4-triazol-4-yl or pyrazol-1-yl and

$R^3$ represents phenoxy, phenylthio, phenylamino or phenyl-N-alkyl-amino with 1 to 4 carbon atoms in the alkyl part, which are in each case optionally mono- or polysubstituted by identical or different substituents, possible phenyl substituents in each case being: halogen, alkyl with 1 to 4 carbon atoms, alkoxy and alkylthio with in each case 1 to 4 carbon atoms; halogenoalkyl, halogenoalkoxy and halogenoalkylthio with in each case 1 to 4 carbon atoms and 1 to 5 identical or different halogen atoms, such as fluorine and chlorine atoms, cycloalkyl with 5 or 6 carbon atoms, nitro, cyano, hydroxycarbonyl, alkylcarbonyl or alkoxycarbonyl with in each case 1 to 4 carbon atoms in the alkyl part, phenoxy or phenyl which is optionally mono- or polysubstituted by identical or different substituents from the group comprising halogen, nitro, trifluoromethyl and alkyl, with 1 to 2 carbon atoms, the aldehyde group and the oxime or oxime ether radical, and their physiologically tolerated acid addition salts.

2. Substituted tert.-butanol derivatives of the general formula (I)

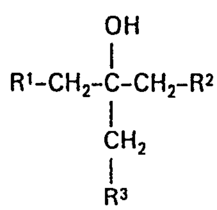

(I)

in which

$R^1$ and $R^2$ are identical or different and represent imidazol-1-yl; 1,2,4-triazol-1-yl; 1,2,4-triazol-4-yl or pyrazol-1-yl and

$R^3$ represents phenoxy, phenylthio, phenylamino or phenyl-N-alkyl-amino with 1 to 2 carbon atoms in the alkyl part, which are in each case optionally mono- to trisubstituted by identical or different substituents, possible phenyl substituents

in each case being: fluorine, chlorine, bromine, methyl, ethyl, isopropyl, tert.-butyl, 2-methyl-but-2-yl, methoxy, ethoxy, methylthio, trifluoromethyl, trifluoromethoxy, trifluoromethylthio, cyclohexyl, nitro, cyano, hydroxycarbonyl, methylcarbonyl, methoxycarbonyl, ethoxycarbonyl, phenoxy and phenyl which are in each case optionally mono- to trisubstituted by identical or different substituents from the group comprising chlorine, nitro, trifluoromethyl and methyl, the aldehyde group, hydroxyiminoethyl and methoxyiminomethyl.

3. 2-(4-Chlorophenoxymethyl)-1,3-di-(1,2,4-triazol-1-yl)-2-hydroxypropane.

4. 2-(2,4-Dichlorophenoxymethyl)-1,3-di-(1,2,4-triazol-1-yl)-2-hydroxypropane.

5. 2-(2,4-Dichlorophenoxymethyl)-2-hydroxy-1-(1,2,4-triazol-1-yl)-3-(1,2,4-triazol-4-yl)-propane.

6. 2-(4-Chlorophenylthiomethyl)-1,3-di-(1,2,4-triazol-1-yl)-2-hydroxypropane.

7. 1,2-Di-(1,2,4-triazol-1-yl)-3-(4-fluoro-phenoxy)-2-hydroxypropane.

8. 1,2-Di-(1,2,4-triazol-1-yl)-3-(4-phenylphenoxy)-2-hydroxypropane.

9. Substituted tert.-butanol derivatives according to Claim 1 for combating mycoses.

10. Substituted tert.-butanol derivatives according to Claims 2 to 8 for combating mycoses.

11. Process for the preparation of substituted tert.-butanol derivatives of the general formula

$$\begin{array}{c} OH \\ | \\ R^1-CH_2-C-CH_2-R^2 \\ | \\ CH_2 \\ | \\ R^3 \end{array} \qquad (I)$$

in which

$R^1$ and $R^2$ are identical or different and represent imidazol-1-yl, 1,2,4-triazol-1-yl; 1,2,4-triazol-4-yl or pyrazol-1-yl and

$R^3$ represents phenoxy, phenylthio, phenylamino or phenyl-N-alkyl-amino with 1 to 4 carbon atoms in the alkyl part, which are in each case optionally mono- or polysubstituted by identical or different substituents, possible phenyl substituents in each case being: halogen, alkyl with 1 to 4 carbon atoms, alkoxy and alkylthio with in each case 1 to 4 carbon atoms; halogenoalkyl, halogenoalkoxy and halogenoalkylthio with in each case 1 to 4 carbon atoms and 1 to 5 identical or different halogen atoms, such as fluorine and chlorine atoms, cycloalkyl with 5 or 6 carbon atoms, nitro, cyano, hydroxycarbonyl, alkylcarbonyl or alkoxycarbonyl with in each case 1 to 4 carbon atoms in the alkyl part, phenoxy or phenyl which is optionally mono- or polysubstituted by identical or different substituents from the group comprising halogen, nitro, trifluoromethyl and alkyl, with 1 to 2 carbon atoms, the aldehyde group and the oxime or oxime ether radical and/or their physiologically tolerated acid addition salts,

characterised in that a nucleophile of the formula (II)

$$R^1-H \qquad (II)$$

in which

$R^1$ has the abovementioned meaning,

a) is reacted with 2-halogenomethyloxiranes of the formula (IV)

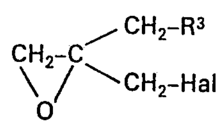

(IV)

in which

$R^3$ has the abovementioned meaning and Hal represents halogen,

in the presence of a diluent and in the presence of an acid-binding agent, or

b) is reacted with substituted oxiranes of the formula (V)

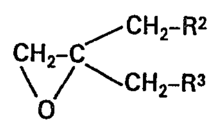

(V)

in which

$R^2$ and $R^3$ have the abovementioned meaning, in the presence of a diluent and in the presence of an acid-binding agent.

12. Medicaments, characterised in that they contain at least one substituted tert.-butanol derivative of the formula (I) in Claim 1.

13. Process for the preparation of antimycotic agents, characterised in that substituted tert.-butanol derivatives according to Claim 1 are mixed with inert, non-toxic, pharmaceutically suitable excipients.

14. Substituted tert.-butanol derivatives according to Claim 1 for use in combating mycoses.